# EUROPEAN PATENT APPLICATION

(11) **EP 4 656 730 A1**
(43) Date of publication of application: **03.12.2025**
(21) Application number: 23918078.9
(22) Date of filing: 14.07.2023
(51) Int. Cl.: C12P 7/44, C12P 7/56

(54) **METHOD FOR PRODUCING POLYLACTIC ACID USING WHEY OBTAINED FROM THE PRODUCTION OF CHEESE**

(30) Priority: 24.01.2023 CL 2023229
(71) Applicant: Plantbased Biotech, S.L., 15707 Santiago de Compostela, La Coruña (ES)
(72) Inventor: PINO SAAVEDRA, Iván, Isla Teja Valdivia (CL)
(74) Representative: Manuel Illescas y Asociados, S.L.U.
(86) International application number: PCT/CL2023/050057
(87) International publication number: WO 2024/156067

(57) **Abstract**

The invention relates to a process for producing polylactic acid (PLA), by means of bacterial fermentation using whey obtained from the production of cheese as the raw material, said process comprising at least the following steps: a) centrifuging the whey at 8000G for 10 minutes; b) quantifying the sugars and proteins; c) recording the density and pH; d) adding an inoculum of the strains *Lactobacillus delbrueckii subsp bulgaricus* and *Streptococcus thermophilus* in an approximate concentration of 75 mg L⁻¹; e) anaerobically growing at 40 ⁰C with stirring at between 200 - 500 rpm for 24 - 96 hours; f) identifying the lactic acid; and g) preparing the PLA, via isolation and purification processes, using physical-mechanical methods such as centrifugation and filtration. The invention also relates to the use of the process to valorize discarded whey from the cheese industry.

## Description

### FIELD OF THE INVENTION

The technology is part of the technological applications related to the use of industrial waste, especially from the dairy industry according to the objectives of the international sustainability guidelines for the production of polylactic acid (PLA), constituting a new, alternative raw material for the production of bioplastics, mainly from sources other than corn.

### STATE OF THE ART

Plastic pollution is a global problem. Approximately 7,000 million of the 9,200 million tons of plastic produced between 1950 and 2017 became plastic waste which ended up in landfills or was dumped.

In this context, it is known that every minute more than a million plastic bags are used with an average "useful life" of only 15 minutes. In turn every year 500 billion plastic bags are used and that only includes single use plastic bags. Only 9% of the nine billion tons of all plastics that have been produced in the world have been recycled. Most end up in landfills, rubbish dumps or in the natural environment. It is expected that by 2025 the ocean will contain one ton of plastic for every three tons of fish and, by 2050, more plastics than fish (by weight).

Plastic pollution can alter habitats and natural processes, reducing the ecosystems' capacity to adapt to climate change, directly affecting the livelihoods of millions of people, their food production capacity and their social well-being.

An alternative to commonly used plastics are biodegradable polymers. These are classified as natural polymers (cellulose, starch and proteins); modified natural polymers (cellulose acetate and polyalkanes); composites combining biodegradable particles (starch, regenerated cellulose or natural rubbers that are combined with synthetic polymers); synthetic polymers (some polyesters, polyesteramides and polyurethanes).

Polylactic acid (PLA) is a thermoplastic biopolymer whose precursor molecule is lactic acid, a natural product obtained from the fermentation of renewable sources. Most of the PLA used worldwide is made from corn (to make 1 kg of PLA, 2.65 kg of corn are needed), competing for an important resource for human food.

The production of biodegradable plastics (bags, packaging, others) recurrently uses polylactic acid (PLA) as raw material (30%-45%), due to its similarity to PET, used in the manufacture of traditional plastics. The large suppliers that market PLA import it from the United States, particularly from the largest producer in the world, NatureWorks. Currently, there are no companies in the domestic market dedicated to the production of this raw material.

Moreover, the national cheese industry is the most important in the production of dairy products. According to ODEPA, just in 2018, cheese production accounted for 37% of total dairy products, followed by powdered milk, fluid milk, yoghurt, and cheese with 27%, 15%, 9%, and 4%, respectively.

This is reflected in the consumption of cheese, with 11.1 kg per capita, the highest in LATAM. This important demand has led to the growth of the national cheese industry, mainly in the regions of Los Ríos and Los Lagos, which grew by 64% just between 2011 and 2016, from US$1,449.6 to US$2,378.9 million.

This growth brings a major challenge, related to sustainability in the cheese production process. In this process, cheese whey is obtained as a by-product, which mainly contains proteins, in addition to lactose, fat and minerals. It is estimated that, for each kilogram of cheese, 10 litres of milk are required, generating 9 litres of whey, that is, it represents about 90% of the milk used, and its annual production is estimated at 800 million litres of whey annually. This effluent is a pollutant due to the high load of BOD and COD making its disposal complex, necessarily requiring treatment and/or ideally adding value through the concept of circularity, entering the waste into new production cycles.

Some technologies that have tried to resolve this problem are:
Patent application US 2006/0036062 presents a Process of producing lactic acid from waste from the agricultural industry, mainly from cane bagasse molasses, corn syrup, sugar juice from cane or beet. The Process has a fermentation step using *Lactobacillus* strains.
Patent application US 2016/0289449 presents a process for producing a bioplastic and the bioplastic, the main components used are protein and a plasticiser. The protein, which corresponds to 5-95% of the bioplastic can come from soy, Zein (corn protein), albumen, whey protein, and combinations thereof.
Patent application WO2010/103548 discloses a mutated strain of *Bacillus coagulans* and its use for the production of lactic acid from nutrient-rich cultures, comprising a carbon source, a nitrogen source; micronutrients and minerals. Where this culture medium can be selected from different sources including Whey.

Despite these new technologies, it is still necessary to provide a new, alternative raw material for the production of PLA, to be used in the production of bioplastics, mainly sources other than corn, so that this is preferably used in food.

The use of renewable sources from any industry that are considered as waste is currently one of the objectives of the International sustainability guidelines, which is why processes that use these wastes are necessary to make use of them in an industry as different from the original one as the manufacture of bioplastics.

### BRIEF DESCRIPTION OF THE FIGURES

**Figure 1****:** Photographic record of samples after the centrifugation process to separate precipitates and fats.
**Figure 2****:** Protein content per present in Vat and Tank samples at day 0, before and after centrifugation.
**Figure 3****:** Protein content in cheese whey samples from Vat and Tank with 0.3 and 7 days of storage.
**Figure 4****:** Protein content in cheese whey samples from Vat and Tank with 3 and 7 days, with and without pasteurisation.
**Figure 5** Reducing sugar content in Vat and Tank whey samples with 3 and 7 days of refrigeration storage, using lactose as a reference.
**Figure 6****:** Lactic acid content recorded after culturing cheese whey from vats, with each lactic strain, at 37°C for 24 hours under anaerobic conditions, with stirring at 175 rpm.
**Figure 7****:** Lactic acid content recorded in the different treatments for vat whey samples.
**Figure 8****:** Lactic acid content recorded in the different treatments for tank whey samples.
**Figure 9****:** Concentration of lactic acid, glucose, lactose and galactose during the period of culture of cheese whey in bioreactor with the bacterium L. *bulgaricus.*
**Figure 10****:** Concentration of lactic acid, glucose, lactose and galactose during the period of culture of cheese whey in a bioreactor with the bacterium *S. thermophilus.*
**Figure 11****:** Concentration of lactic acid, glucose, lactose and galactose during the period of culture of cheese whey powder (Lactocolun) in a bioreactor with the bacterium L. *bulgaricus.*
**Figure 12****:** Chromatogram of the sample of lactic acid recovered from fermented cheese whey. Chromatogram corresponding to the initial sample of the culture and the sample obtained after the recovery process.
**Figure 13****:** FTIR transmittance spectra of lactic acid samples, commercial PLA and PLA Process obtained in the laboratory.

### DESCRIPTION OF THE INVENTION

A process for producing polylactic acid (PLA) is presented, by means of bacterial fermentation, using whey obtained from the production of cheese as raw material.

The whey used can be obtained immediately after curdling the cheese or stored for at least 24 hours after processing.

The process comprises at least the following steps:
a) Centrifuging the whey to discard precipitates and fats;
b) Quantifying the sugars and proteins, to determine lactic acid productivities;
c) Recording the density and pH, necessary as a quality control of the whey that is entered into the process, if necessary, the pH should be adjusted between 5.8 ~ 6.5;
d) Adding an inoculum of lactic strains selected from: *Lactobacillus delbrueckii, Lactobacillus casei, Staphylococcus aureus, Lactobacillus bulgaricus, Lactobacillus leichmannii, and* strains of the genera Pediococcus and *Streptococcus,* in an approximate concentration of 75 mg L⁻¹; anaerobically growing same at 40°C with stirring at between 200-500 rpm for 24-96 hours, depending on the quality of the whey received;
e) Identifying the lactic acid by HPLC quantification with refractive index detector and a column for quantitative determination of organic acids;
f) Purifying the lactic acid from cheese whey cultured with lactic bacteria, by centrifugation of the fermented whey, tangential filtration of the supernatant, acidification, clarification of the filtrate with activated carbon, concentration of the clarified portion, crystallisation of impurities and final filtration.
g) Polymerisation of lactic acid using ring-opening polymerisation (ROP) Processology with SnCl2 as catalyst and xylene as solvent.

Since the different samples contain a considerable amount of agglomerates and fats, these must be separated from the whey prior to fermentation, via centrifugation. It is important to quantify sugars and proteins at the start of the process, due to the differences between the values of the whey just received and that which is stored, for example, the protein content of the whey at the time of receipt of the samples is close to 1000 mg/L, but during storage after 3 and 7 days, the content drops to a minimum close to 700 mg/L, there being no differences between the vat and tank samples. The same happens in the case of sugars where the sugar content is in a range of 28.48 - 41.88 ± 6 g/L, which is why storage for long periods of time (more than 1 week) should be avoided, since despite the whey being kept refrigerated, there is an initial bacterial load that contributes to the degradation of simple sugars such as glucose and lactose, favouring the presence of a basal content of lactic acid (15-30 g/L).

Preferably, it is recommended to use the bacterial strain *S. thermophilus,* which allowed obtaining higher concentrations of lactic acid, compared to cultures with *L. bulgaricus* and other bacteria.

Optionally, this process can be carried out using cheese whey powder, adjusting the culture to 120 g/L of initial sugars, it is possible to produce lactic acid, reaching 40 g/L after 96 hours of growth.

With this process it is possible to recover 99% of the lactic acid present in a culture of cheese whey powder inoculated with *bulgaricus* following the recovery sequence.

The production of low molecular weight polylactic acid is performed using the ring-opening polymerisation (ROP) Process, with SnCl2 as catalyst and xylene as solvent, a semi-crystalline product is obtained that can be extruded at a temperature of 125°C. The PLA obtained has chemical properties similar to commercial PLA, according to the FTIR spectra analysed.

The advantages of using this process involve addressing a key market opportunity, incorporating into the domestic market the production of PLA for the production of various biodegradable plastic products, utilising discarded whey from the domestic cheese industry, which represents about 90% of the milk used, estimating an annual production of 800 million litres of whey annually in our country.

Additionally, and contrary to current practice, this process does not use food ingredients for the production of PLA. This Process considers the use of a by-product or waste product that is currently sold mainly for animal feed, at low prices, and even translates into losses as it represents an important polluting effluent that must be treated.

### APPLICATION EXAMPLES

### Example 1: Characterisation of the content of reducing sugars and proteins present in cheese whey

Samples of cheese whey from a cheese company located in the Los Ríos Region were analysed. Two types of samples were analysed 1) Vat or first curd: They correspond to samples taken immediately after the processing or curdling of the cheese; 2) Tank: They correspond to samples stored at room temperature (24-48 hours) after the cheese has been made.

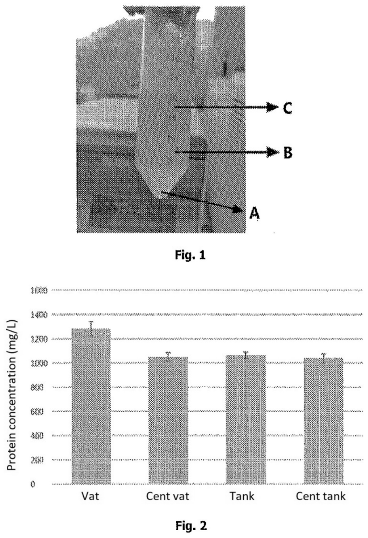

For both cases, a mechanical post-treatment of centrifugation of the samples was considered, due to the presence of precipitates and fat, which could interfere with the quantification results, the samples were centrifuged at 8000G for 10 minutes. In figure 1, an example of the phase separation of a sample after centrifugation can be seen, leaving at the bottom a pellet with a large part of the precipitates (A), an intermediate layer of supernatant where the sugars of interest (B) are found and an upper layer that mainly comprises the fats present in the whey (C).

To determine whether the storage time of the whey, prior to fermentation with lactic bacteria, has any effect on the content of sugars and proteins present therein, the samples received were analysed at 3 storage times, that is: immediately (day 0) and after 3 and 7 days of refrigeration at 4°C, respectively. Additionally, each sample was analysed after sterilisation by pasteurisation, subjecting the samples to a treatment of 80°C for 30 minutes, in order to identify the effect of sterilisation on the sugar and protein content.

### a) Protein quantification

The proteins present in the vat and tank whey samples were measured using the Bradford Process. To this end, a calibration curve was performed using Bovine Serum Albumin (BSA) protein as standard. To produce the curve, first, 7 serial dilutions were prepared with the following BSA concentrations: 2; 1.5; 0.75; 0.5; 0.25; 0.125 and 0 mg/mL. Each sample was treated with Bradford's reagent (Bio-Rad code 5000205) following the supplier's instructions and subsequently its absorbance (595 nm) was recorded using a Nanophotometer Model NP80 spectrophotometer. Using the generated calibration curve, the total proteins of each sample were quantified following the same Processology.

In parallel, the integrity of the proteins present in the samples was observed using the SDS-PAGE Process

The total protein content detected in the samples at day 0 are shown in Table 1. It is noted that each sample was analysed before and after being centrifuged.

**Table 1: Protein quantification results using the Bradford Process at day 0**

| **Sample** | **Average protein content (mg/L)** |
|---|---|
| Vat | 1283.46 |
| Vat post-centrifugation | 1050.13 |
| Tank | 1062.21 |
| Post-centrifugation tank | 1036.79 |

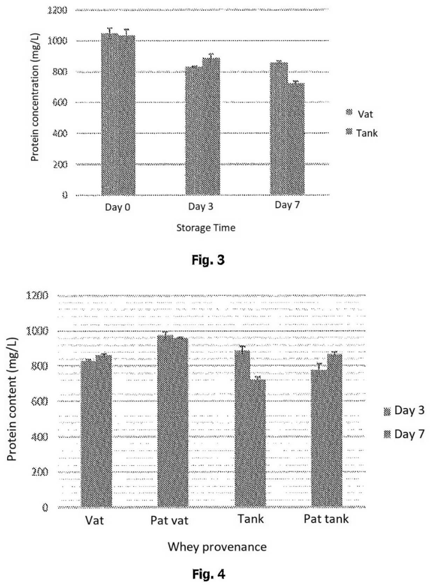

As seen in figure 2, no major differences in protein content were observed between the Vat and Tank samples at day 0 after the centrifugation process. A slight decrease was observed in the Vat sample probably due to the precipitation of proteins in conjunction with other agglomerates present in the sample once centrifuged. Given these results, all protein analyses performed hereafter considered a pre-quantification centrifugation. Regarding the protein content once the samples have been stored for 3 or 7 days, it is observed that in all cases the protein content is slightly lower than that recorded on day 0. At day zero the protein content in both centrifuged vat and tank samples were above 1000 mg/L, while samples stored for 3 -7 days are below this value, in a range of 722 to 887 g/L (Table 2, Figure 3).

**Table 2: Average protein content in samples stored under refrigeration.**

| **Sample** | **Average protein content (mg/L)** |
|---|---|
| Vat day 0 | 1050.12 |
| Tank day 0 | 1036.79 |
| Vat day 3 | 831.37 |
| Vat day 7 | 860.96 |
| Tank day 3 | 887.21 |
| Tank day 7 | 722.21 |

It is important to note that after the pasteurisation process, some samples showed greater turbidity, as observed in the Vat and Tank samples, on days 3 and 7 post-pasteurisation. This turbidity affects the absorbance of the samples and therefore the quantifications performed, showing results that could be erroneous, so the samples were re-centrifuged at 8000G x 10 minutes. Once re-centrifuged, the protein content of each sample was re-recorded (Table 3).

**Table 3: Results of protein quantification using the Bradford Process at days 3 and 7 of storage, with and without pasteurisation.**

| | **VAT** | **PAST VAT** | **TANK** | **PAST TANK** |
|---|---|---|---|---|
| Day 3 | 831.37 ± 4.33 g/L | 972.21 ± 24.66 g/L | 887.21 ± 25.38 g/L | 775.96 ± 35.63 g/L |
| Day 7 | 860.96 ± 8.32 g/L | 959.71 ±3.15 g/L | 722.21 ±15.07 g/L | 867.21 ±13.13 g/L |

In general, no significant differences were observed between pasteurised and unpasteurised samples at days 3 and 7, except for the unpasteurised tank sample at day 7, where a decrease of 165 g/L was recorded (Figure 4).

With the results obtained, it can be concluded that the storage time up to 7 days slightly affects the content of proteins present in the whey. This content is also not significantly affected when applying a pasteurised sterilisation treatment.

### b) Quantifying the reducing sugars

Reducing sugars were quantified using the 3,5 dinitrosalicylic acid (DNS) Process, following the protocol described by Bello, Bocourt and Maqueira (2006). First, a calibration curve was produced using lactose as a standard for reducing sugars. The calibration curves were constructed by performing serial dilutions with the following lactose concentrations: 0.5, 0.25, 0.1, 0.01 and 0 g/L, which were treated with DNS according to the protocol of Bello, Bocourt and Maqueira (2006). At each point, the absorbance (540 nm) was recorded using a Dynamic Spectrophotometer model HALO DB-20. Once the curve was constructed, each sample was centrifuged at 8000G for 10 minutes, discarding the precipitates and fats present in the sample and recovering the supernatant, which was treated with DNS following the same Processology described for the construction of the calibration curve. The absorbance (540 nm) of each sample was recorded and the content of reducing sugars present in the sample was determined using the curve.

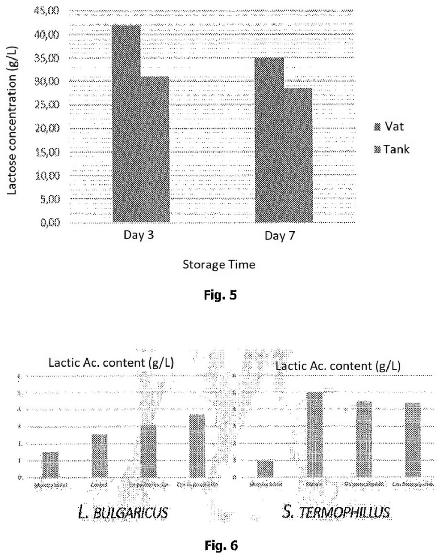

Quantification of reducing sugars, using lactose as a reference, demonstrated that the content of reducing sugars is lower in the tank samples, compared to the vat samples, as shown in Figure 5. On day 3 the lactose content in the vat was 41.88 g/L, while in the tank it was 30.04 g/L. On day 7, the Vat whey sample had 35.04 g/L lactose, while the Tank sample had 28.48 g/L. These results may be related to the action of microorganisms present in the whey, which use these sugars for their development, including lactic bacteria already present in the whey prior to the fermentation process. The importance of whey storage conditions to prevent sugar depletion before whey enters the fermentation process is therefore highlighted.

**Table 4: Sample Identification**

| **Determination** | **Unit** | **Vat Sample** | **Tank Sample** |
|---|---|---|---|
| Energy | Kcal | 19.9 | 16.9 |
| Moisture | % | 94.4 | 95.05 |
| Protein (N 6.25) | % | 0.7 | 0.61 |
| Total Fats⁽¹⁾ | % | 0.05 | 0.05 |
| Crude Fibre | % | 0.26 | 0.36 |
| Carbohydrates⁽²⁾ | % | 4.2 | 3.5 |
| Ash | % | 0.44 | 0.41 |
| Total Sugars ⁽³⁾ | g/100 g | 2.2 | 1.8 |

| | | | |
|---|---|---|---|
| ⁽¹⁾ Gerber Process according to Kehe (≥0.05%) ⁽²⁾ Available CH ⁽³⁾ Total Sugars: Munson and Walker Process % = grams in 100 grams of sample CV% < 0.4% | | | |

The reducing sugar content decreases significantly when keeping the whey stored in tanks at room temperature after cheese production. However, when keeping the whey refrigerated for 3 to 7 days, no significant decreases in reducing sugar content are observed.

### c) Recording the density and pH:

The density recorded in the Vat and Tank samples was 1.025 g/mL and 1.020 g/mL, respectively. According to these results, there is a minor difference of only 0.005 g/L in the density of both samples. In contrast, pH measurements indicate that there is a considerable difference between the Vat and Tank samples. The Vat samples recorded an average pH of 6.45, while the Tank samples recorded an average pH of 3.67. This difference is important because it shows that whey can acidify quickly after being stored in the tank. The pH significantly influences characteristics of the growth of lactic bacteria, such as the maximum concentration of biomass, the specific growth rate, the fermentation time, the consumption or growth of sugar and the product yields. Various studies have determined the optimal pH ranges for various lactic acid-producing bacteria. In the case of bacteria considered *Streptococcus thermophilus* which has an optimal pH close to 6.5, while for *Lactobacillus bulgaricus* the optimal pH is in a range of 5.8 to 6 (Rault, Bouix and Beal 2009). It will be important, therefore, to adjust the pH of the whey that enters the subsequent fermentation processes in order to maintain it in the optimal ranges described for the bacteria to be used.

### Example 2: Evaluation of production yield and selection of lactic acid producing strain(s)

The lactic acid production yield of both bacteria will be evaluated by taking samples from Vat and Tank previously centrifuged at 8000G for 10 minutes, pasteurised samples (80°C for 30 min) and unpasteurised samples will be evaluated. The strains *Lactobacillus delbrueckii subsp bulgaricus (Lb-12) and Streptococcus thermophilus (STI-12)* of CHR-HANSEN will be analysed separately and together.

The experimental design was proposed to verify the effect of the sterilisation of the whey prior to culture with the lactic strains, which were inoculated in vat and tank whey samples, evaluated individually and jointly, recording the lactic acid yield in each case.

Preparation of samples for inoculation: The samples were centrifuged at 8000G for 10 minutes, discarding precipitates and fats. The supernatant obtained was further transferred to a decantation flask to make a final separation of residues of fatty matter present in the sample.

Inoculation of cheese whey with lactic bacteria: First, pre-inocula *L*. *bulgaricus and S. thermophilus* a spatula tip (equivalent to two granules of lyophilised bacteria) were prepared to 120 mL of sterile RMS (Dilaco) medium, prepared according to the supplier's indications, in a 250 mL Erlenmeyer flask. Pre-inocula were grown at 37°C with stirring at 175 rpm for 16 hours.

Once the pre-inocula were prepared, 120 mL of whey was inoculated with 1 mL of pre-inoculum for the case of cultures with the bacteria individually, or with 500 µL of each pre-inoculum for the treatments with both bacteria. Additionally, a control sample was prepared without any inoculum.

The flasks of each treatment were incubated in a GASPAK EZ STD Incubation Container system, which also included a CO2 generator sachet to ensure the anaerobic conditions of the culture. After the end of the culture period, a 2 mL sample was taken from each flask, which was used to quantify, via HPLC, the lactic acid content present in the sample.

The amount of lactic acid was quantified by culturing samples from the vat and tank, with and without pasteurisation, using the previously mentioned lactic strains individually or together.

The results of the vat samples cultured with each strain individually, where a significantly higher LA content is observed after culture with *S*. *thermophilus,* compared to *L*. *bulgaricus* (Figure 6).

Evaluation of the lactic acid content recorded in vat samples: In the case of the Vat samples, a considerable increase in the lactic acid content was observed at all points, with respect to the initial sample, both in the cultures with each bacterium individually, as well as with the mix of bacteria. The control samples, although not inoculated, also showed an increase in the lactic acid content, which is probably due to the presence of lactic bacteria already present in the initial samples, which, under the appropriate anaerobic conditions, also produce lactic acid.

When comparing the lactic acid content obtained using each bacterium separately, it is observed that in the case of *L*. *bulgaricus* there is a considerable increase in lactic acid after inoculation, which is even greater in the pasteurised samples. On the contrary, in the case of *S*. *thermophilus,* although a considerable increase is observed with respect to the initial sample, there were no significant differences between the different treatments. When using a mix of lactic acid bacteria, the inoculated samples showed an increase compared to the control without inoculum, without showing major differences between pasteurised and unpasteurised samples (see Tables 5 and 6).

These results show, in general, that *S*. *thermophilus* performs better under the conditions evaluated. Moreover, the pasteurisation process has no significant effect on the final lactic acid content present in the samples after culture.

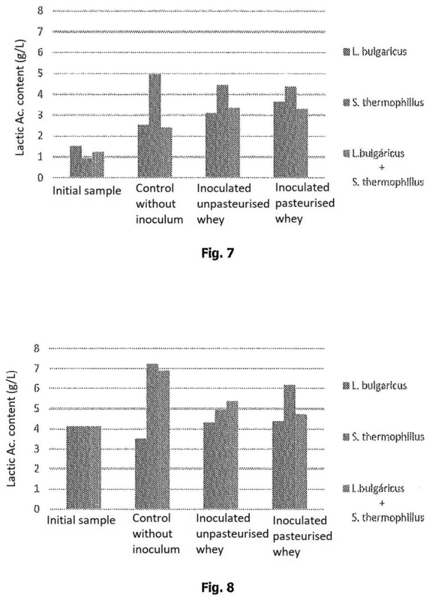

These observations are presented graphically in Figure 7 and 8 for whey samples from Vat and whey samples from Tank.

**Table 5: Production of lactic acid (g/L) in vat samples treated with lactic acid bacteria.**

| | *L. bulgaricus* | *S. thermophilus* | *L. bulgaricus + S. thermophilus* |
|---|---|---|---|
| Initial sample | 1.51 | 0.923 | 1.2165 |
| Control without inoculum | 2.517 | 4.973 | 2.402 |
| Inoculated unpasteurised whey | 3.089 | 4.446 | 3.353 |
| Inoculated pasteurised whey | 3.658 | 4.365 | 3.317 |

**Table 6: Production of lactic acid (g/L) in tank samples treated with lactic bacteria.**

| | *L. bulgaricus* | *S. thermophilus* | *L. bulgaricus + S. thermophilus* |
|---|---|---|---|
| Initial sample | 4.109 | 4.109 | 4.109 |
| Control without inoculum | 3.505 | 7.242 | 6.889 |
| Inoculated unpasteurised whey | 4.289 | 4.933 | 5.361 |
| Inoculated pasteurised whey | 4.372 | 6.182 | 4.718 |

### Example 3: Optimisation of the lactic acid production process from the fermentation of cheese whey

The cultures were performed in a bioreactor with controlled conditions of pH, temperature, stirring and CO₂. Additionally, to optimise the conversion to lactic acid, lactase and proteases were added to the culture.

Given the above results, it was decided that from now on the tests will be carried out only with tank samples, since it is a process that has a greater presence of lactic acid naturally and also since it is the conditions closest to the industry, which favour a future scaling stage.

Approximately 40 L of cheese whey separated into 2 drums was worked. One drum contained whey collected at 12:30 pm while the second contained whey collected at 4:30 pm. A sample from each drum was collected at the time of receipt, recording the pH, sugar content and basal lactic acid concentration following the same Processology described previously.

The contents of both drums were centrifuged following the protocol previously described. With the centrifuged whey, cultures will be made in bioreactors following the experimental design:
a) Pre-treatment: centrifugation; spray drying and pasteurisation.
b) Culture: in 3 L reactor (duplicate), pH control, Temperature, stirring, CO₂, lactase and protease;
c) Analysis: Initial sugar quantification (DNS and HPLC)
   Post-culture Lactic Acid Quantification (HPLC)

Although in the previous activities it was observed that in most cases pasteurisation does not have a significant effect on the final lactic acid content, in the new design this stage was maintained considering that we will work only with tank samples that could contain a significant bacterial load and that could affect the final results.

The pH of the drum sample collected at 12:30 pm was 6.29, while the 4:30 pm sample had a pH of 3.14. These results, as well as those obtained in the first activities, show the importance of making a pH adjustment prior to the cultures, considering that in just 4 hours there is already a significant decrease in pH that could affect the growth and activity of the lactic acid bacteria to be used.

Using lactose as a reference, the content of reducing sugars present in the 12:30 pm drum was 56.38 g/L, while for the 4:30 pm sample, a content of 37.10 g/L was recorded. These results are consistent with the increase in pH observed, which indicates that part of the sugars have already begun to be consumed by bacteria basally present in the whey during the hours of storage, generating lactic acid and consequently an increase in the acidity of the sample.

Subsequently, it continued with 2.5 L of cheese whey, previously centrifuged and sterilised, which were cultured in an INFORS-BT bioreactor, for each bacterial strain to be evaluated. The initial pH of the culture was adjusted to 5.8 according to what is suggested in the literature. Once the pH was adjusted and the culture temperature reached (38°C), the different lyophilised lactic acid bacteria were added, according to the supplier's (DILACO) instructions. During the culture, the parameters of pH, temperature, 02 concentration and stirring were kept under control. Culture samples and 1-2 hour time intervals were taken for 56 hours by recording the lactic acid content at each point by liquid chromatography (HPLC). In addition, the sugars glucose, lactose and galactose were quantified at each point.

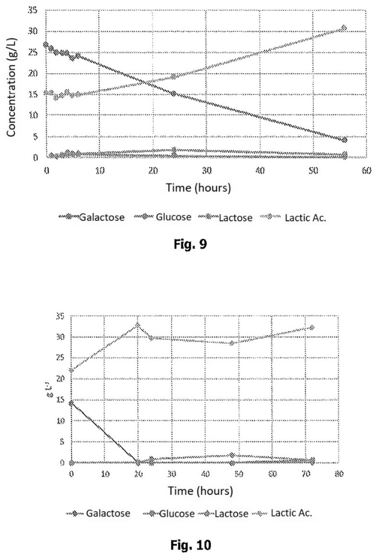

Table 7 summarises the results obtained during whey culture in a bioreactor with the bacterium *L*. *bulgaricus.* It is observed that, by maintaining the cultures for 56 hours, the lactic acid content manages to double, from an initial concentration of 15.27 g/L to a final concentration of 30.694 g/L, indicating a transformation of 73% of the sugars quantified into lactic acid. It is important to note that the initial quantifications of sugars such as glucose and lactose are low, that is, less than 1 g/L, which is probably related to the cold storage time of the sample prior to the execution of the assay, during which, as previously mentioned, the basic bacteria of the whey rapidly consume these sugars. However, in real conditions, these levels could be higher if shorter storage times are considered. In contrast, galactose maintains higher concentrations at the first sampling points, which decrease significantly after at least 2 days of culture. Making shorter cultures under these conditions would therefore imply losing much of the potential of galactose to be transformed into lactic acid by the bacteria used. The same results are presented graphically in figure 9.

**Table 7: Quantification of sugars and lactic acid during cheese whey culture with L. bulgaricus in bioreactor**

| **Time (hours)** | **Concentration (g/L)** | | | | **Total** | **Balance** |
|---|---|---|---|---|---|---|
| | **Galactose** | **Glucose** | **Lactose** | **Lactic Ac.** | | |
| 0 | 26.798 | | | 15.227 | 42.03 | 36.23% |
| 1 | 25.891 | | 0.382 | 15.343 | 41.62 | 36.51% |
| 2 | 24.967 | | 0.289 | 14.18 | 39.44 | 33.74% |
| 3 | 24.824 | 0.356 | 0.541 | 14.59 | 40.31 | 34.72% |
| 4 | 24.682 | 0.769 | 1.098 | 15.545 | 42.09 | 36.99% |
| 5 | 23.54 | 0.676 | 0.785 | 14.635 | 39.64 | 34.82% |
| 6 | 24.102 | 0.822 | 0.947 | 14.926 | 40.80 | 35.52% |
| 24 | 15.107 | 0.381 | 1.804 | 19.057 | 36.35 | 45.35% |
| 56 | 3.9656 | 0 | 0.723 | 30.694 | 35.38 | 73.04% |

Given the results obtained for *L*. *bulgaricus,* in order to optimise, the culture of S. *thermophilus* was performed under the same conditions; however, the initial inoculum was increased to twice the supplier's suggestion, that is, 150 mg in 2.5 L of whey (initial culture concentration = 60 mg/L). On this occasion, the samples were taken after longer periods of culture time, considering that in the first hours there were no major variations in the lactic acid content. The culture was maintained under the same conditions for 72 hours. As in the culture with *L*. *bulgaricus,* there was already an initial content of lactic acid in the whey (22 g/L), representing a 60% conversion of sugars present in the sample. The initial lactose and glucose levels were again considerably low (0-1.8 g/L), indicating that they are rapidly consumed during whey storage, leaving mostly galactose as a source for the generation of lactic acid. Unlike the culture of *L*. *bulgaricus,* in which the level of galactose was almost completely reduced after 56 hours of culture, with *S. thermophilus* we observed that after 24 hours there are no detectable levels of galactose, achieving close levels of lactic acid production that are around 30 g/L. These results are presented in Table 8 and are graphically summarised in Figure 10.

**Table 8: Quantification of sugars and lactic acid during the culture of cheese whey with S. thermophilus in a bioreactor.**

| **Time** | **Concentration (g/L)** | | | | **Total** | **Balance** |
|---|---|---|---|---|---|---|
| | **Galactose** | **Glucose** | **Lactose** | **Lactic Ac.** | | |
| 0 | 14.21 | 0 | 0 | 22.061 | 36.27 | 60.82% |
| 20 | 0.118 | 0 | 0.17 | 32.78 | 33.07 | 90.38% |
| 24 | 0 | 0 | 0.947 | 29.75 | 30.70 | 82.02% |
| 48 | 0 | 0 | 1.804 | 28.49 | 30.29 | 78.55% |
| 72 | 0.67 | 0.072 | 0.723 | 32.28 | 33.75 | 89.00% |

### Example 4: Purification of lactic acid present in whey from cheese powder fermented with lactic bacteria

Similar to what is described in previous examples, a culture of dry whey powder (spray drying) of commercial origin was performed, where the bioreactor assembly used 155 g of whey powder, which were re-suspended in 2 L of distilled water, equivalent to 120 g/L of total sugars according to nutritional information indicated by the supplier. Prior to the fermentation, a pretreatment was carried out with 20 mL of alkaline protease to hydrolyse proteins present, incubating at 60°C, pH 7.5 for 2 h. Subsequently, pH was lowered with phosphoric acid to pH 6.5 and inoculated with 200 mg of lyophilised culture of *L*. *bulgaricus,* maintaining the culture at 40°C for 96 h, under the same conditions mentioned for previous tests, the quantification data are shown in table 9.

**Table 9: Quantification of sugars and lactic acid during culture of cheese whey powder with L. bulgaricus in bioreactor Concentration (g/L),**

| **Time (hours)** | **Concentration (g/L)** | | | | **Total** | **Balance** |
|---|---|---|---|---|---|---|
| | **Galactose** | **Glucose** | **Lactose** | **Lactic Acid** | | |
| 0 | 46.431 | 6.56 | 8.25 | 0.533 | 61.774 | 0.86% |
| 24 | 34.431 | 5.839 | 8.211 | 8.41 | 56.891 | 14.78% |
| 48 | 20.705 | 9.6 | 14.79 | 23.68 | 68.775 | 34.43% |
| 72 | 14 | 12.3 | 18.6 | 29.2 | 74.1 | 39.41% |
| 96 | 9.779 | 0 | 20.72 | 40.465 | 70.964 | 57.02% |

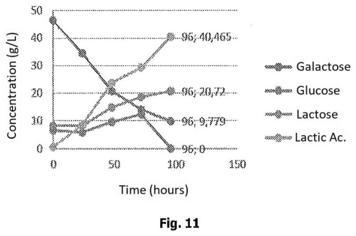

In contrast to previous experiences, this time the basal lactic acid content present in the sample is considerably lower (0.533 g/L), probably due to a shorter storage time prior to drying, a condition under which the production of lactic acid is stopped. When using the whey powder, the culture had to be maintained for a longer time to reach the ranges previously obtained with the whey in liquid format, reaching about 30 g/L after 72 hours of culture. Extending the culture for an additional 24 hours, the concentration increases to 40 g/L, being higher than the values obtained in previous tests, with a balance close to 60%. The same results are presented graphically in figure 11.

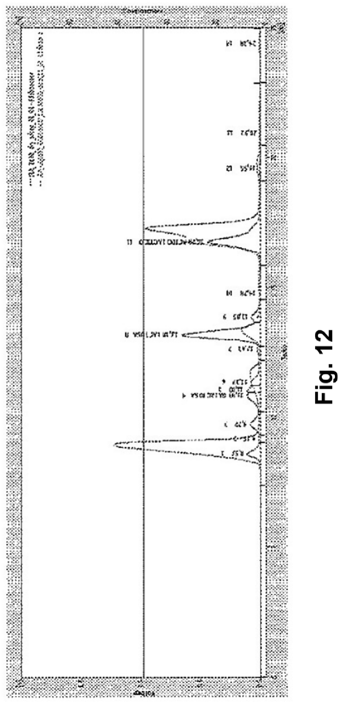

Lactic acid recovery was performed from the last bioreactor culture using cheese whey powder (see act. 2.1 c). First, the biomass was separated from the culture medium with lactic acid using a 30kD Vivaflow^{®} 200 filter (Sartorius), the culture entering the filter by means of a peristaltic pump at a flow of 400 mL per minute, with an outlet pressure of 2.5 bar. The biomass was discarded and the filtrate recovered for the extraction of lactic acid. The filtrate was subsequently acidified by adding H2SO4 until reaching a pH of 2.5, thereby managing to take the calcium lactate present in the culture to lactic acid. This solution was subsequently treated with 5% activated carbon for decolourisation in the batch system, maintaining stirring at 160 rpm for 2 hours. After this period, we filtered through a Whatmann No. 2 paper. The obtained filtrate was concentrated under reduced pressure (60°C, 80 mb). During this process, Impurities present in the solution are crystallised, which were discarded by filtration with Whatmann No. 2, repeating the concentration process until no impurities are observed. The purified lactic acid was analysed by HPLC to verify purity and concentration. After analysing the chromatogram (Figure 12), it was determined that, after the purification process, 99% of the lactic acid present in the sample was recovered.

### Example 5: Polymerisation of lactic acid from the fermentation of cheese whey

Three protocols were analysed to evaluate lactic acid polymerisation. These are the results obtained after the execution of each one:
**Protocol 1:** First, the formation of lactide starts in the presence of sulfuric acid, a reaction that takes 20 h. Subsequently, the ring-opening polymerisation reaction, ROP (Ring-Opening Polymerisation), is carried out using SnCl2 as a catalyst and methane as a solvent. The first 2 hours at 80°C to allow the dehydration of lactic acid, and then raise the temperature to 120°C for 18 h, during which time sulfuric acid is added. Subsequently, for the development of the ROP reaction, the temperature is lowered to 60°C and the catalyst and methanol are added, leaving to react for 24 h.

In the process carried out for this test, it was detected that an excess of sulfuric acid, the high temperatures used in this procedure and some impurities present in the lactic acid, caused a carbonisation of the reaction mixture not being able to synthesise the expected PLA.

**Protocol 2:** The second test was carried out by dehydration, polymerisation, depolymerisation and polymerisation by continuous ROP under reduced pressure. This Process was conducted on a rotary evaporator to control the working temperature and pressure. SnCl2 was used as the catalyst of the ROP reaction. The operating conditions are described in the following table 10.

**Table 10:**

| **Process phase** | **Time (hours)** | **Temperature (°C)** | **Pressure (mbar)** |
|---|---|---|---|
| Dehydration | 1 | 90 | 100 |
| Polymerisation | 6 | 160 | 100 |
| Depolymerisation | 16 | 180 | 100 |
| ROP | 6 | 140 | 100 |

In this case, it was possible to perform the first two phases of the process, however, it was not possible to reach the working temperature, which could be resolved using suitable equipment to reach these temperatures. Likewise, a solid product was obtained at room temperature and liquid at a temperature of 120°C.

**Protocol 3.** Finally, the third test consisted of carrying out a direct ROP reaction using SnCl2 as a catalyst and xylene as a solvent, between 20-30% lactic acid, 60-80% xylene and 0.5-3% SnCl2 were used. The reaction was incubated on a rotary evaporator at 140°C for 30 h. After the reaction, it was washed with methanol to remove excess xylene and left in the oven at 80°C to dry the final product.

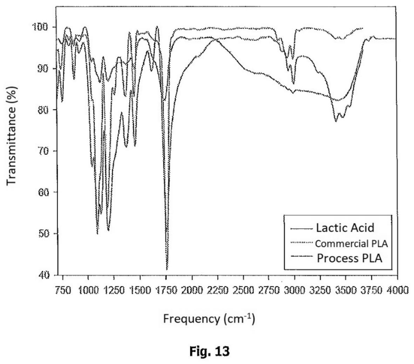

This third protocol was the most successful of those carried out, achieving a semi-crystalline solid product that according to the analyses carried out by FTIR, corresponds to polylactic acid (Figure 13). According to the melting points studied in the laboratory, it would correspond to low molecular weight polymers. According to the infrared analysis, samples of lactic acid obtained from cheese whey, commercial PLA and PLA obtained after the polymerisation of lactic acid obtained in the laboratory were compared. The first difference that can be observed is that the absorption spectrum of lactic acid is very different from that observed in PLA samples. Both PLA samples show a very similar spectrum profile, where the highest absorption peaks are almost identical, indicating the presence of the same functional groups in both compounds. However, there are some peaks of PLA produced in the laboratory wider than the commercial one, which would indicate that they are of low molecular weight and probably some interferent present in the product.

## Claims

1. Process for producing polylactic acid (PLA), by means of bacterial fermentation, **CHARACTERISED in that** it uses whey obtained from the production of cheese as raw material.

2. Process for producing polylactic acid (PLA), by means of bacterial fermentation, according to claim 1, **CHARACTERISED in that** the whey used can be obtained immediately after the curdling of the cheese or stored for at least 24 hours after processing.

3. Process for producing polylactic acid (PLA), by means of bacterial fermentation, according to claim 1, **CHARACTERISED in that** it comprises at least the following steps:
a) Centrifuging the whey at 8000G for 10 minutes;
b) Quantifying the sugars and proteins, to determine lactic acid productivities;
c) Recording the density and pH as a quality control of the whey that is entered into the process;
d) Adding an inoculum of lactic strains selected from: *Lactobacillus delbrueckii, Lactobacillus casei, Staphylococcus aureus, Lactobacillus bulgaricus, Lactobacillus leichmannii,* and strains of the genera *Pediococcus* and *Streptococcus,* in an approximate concentration of 75 mg L⁻¹;
e) Anaerobically growing same at 40°C with stirring at between 200-500 rpm for 24-96 hours;
f) Identifying the lactic acid by HPLC quantification with refractive index detector and a column for quantitative determination of organic acids;
g) Identifying the lactic acid by HPLC quantification with refractive index detector and a column for quantitative determination of organic acids;
h) Purifying the lactic acid from cheese whey grown with lactic bacteria, by centrifugation of the fermented whey, tangential filtration of the supernatant, acidification, clarification of the filtrate with activated charcoal, concentration of the clarified portion, crystallisation of impurities and final filtration; and
i) Polymerisation of lactic acid using ring-opening polymerisation (ROP) Processology with SnCl2 as a catalyst and xylene as a solvent, the conditions used need to be clarified.

4. Process for producing polylactic acid (PLA), by means of bacterial fermentation, according to claim 1, **CHARACTERISED in that** in step c) the pH must be adjusted between 5.8-6.5 if necessary.

5. Process for producing polylactic acid (PLA), by means of bacterial fermentation, according to claim 1, **CHARACTERISED in that** in step e) the culture time is proportional to the amount of proteins and sugars of the samples.

6. Process for producing polylactic acid (PLA), by means of bacterial fermentation, according to claim 1, **CHARACTERISED in that** cheese whey powder can optionally be used, adjusting the culture to 120 g/L of initial sugars.

7. Use of the Process for producing polylactic acid (PLA), by means of bacterial fermentation, according to claim 1, **CHARACTERISED IN THAT** it serves to valorize discarded whey from the cheese industry.
